(19) Europäisches Patentamt
European Patent Office
Office européen des brevets

(11) **EP 3 591 378 B1**

(12) **EUROPÄISCHE PATENTSCHRIFT**

(45) Veröffentlichungstag und Bekanntmachung des Hinweises auf die Patenterteilung:
**18.11.2020   Patentblatt 2020/47**

(51) Int Cl.:
**G01N 21/31** *(2006.01)*      **G01N 33/49** *(2006.01)*

(21) Anmeldenummer: **18181329.6**

(22) Anmeldetag: **03.07.2018**

(54) **VERFAHREN ZUR BESTIMMUNG VON LIPIDEN, HÄMOGLOBIN UND BILIRUBIN IN KÖRPERFLÜSSIGKEITSPROBEN**

METHOD FOR ASSAYING LIPIDS, HAEMOGLOBIN AND BILIRUBIN IN BODY FLUID SAMPLES

PROCÉDÉ DE DÉTERMINATION DES LIPIDES, DE HÉMOGLOBINE ET DE BILIRUBINE DANS DES ÉCHANTILLONS DE LIQUIDE CORPOREL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**08.01.2020   Patentblatt 2020/02**

(73) Patentinhaber: **Siemens Healthcare Diagnostics Products GmbH**
**35041 Marburg (DE)**

(72) Erfinder: **Sass, Karl**
**35274 Kirchhain (DE)**

(56) Entgegenhaltungen:
**EP-A1- 3 051 271     US-A1- 2014 192 342**

EP 3 591 378 B1

**Beschreibung**

Die vorliegende Erfindung liegt auf dem Gebiet der in vitro-Diagnostik und betrifft ein Verfahren und ein automatisches Analysegerät zur quantitativen Bestimmung von Lipiden, Hämoglobin und Bilirubin in Blutserum- und Blutplasmaproben.

Zahlreiche Nachweis- und Analyseverfahren zur Bestimmung physiologischer Parameter in Körperflüssigkeitsproben beruhen auf photometrischen Messprinzipien. Photometrische Verfahren ermöglichen den qualitativen und quantitativen Nachweis von Analyten in flüssigen Proben.

Die Bestimmung klinisch relevanter Parameter, wie zum Beispiel der Konzentration oder der Aktivität eines Analyten erfolgt vielfach, indem ein Aliquot einer Körperflüssigkeit eines Patienten mit einem oder mehreren Testreagenzien in vitro vermischt wird, wodurch eine biochemische Reaktion in Gang gesetzt wird, die eine messbare Veränderung einer optischen Eigenschaft des Testansatzes bewirkt. Die Photometrie untersucht und nutzt die Schwächung eines Lichtstroms beim Durchtritt durch ein absorbierendes und/oder streuendes Medium. Je nach Art der ausgelösten biochemischen oder biophysikalischen Reaktion kommen unterschiedliche photometrische Messverfahren zum Einsatz, die die Messung eines trüben flüssigen Testansatzes ermöglichen.

Hierzu können turbidimetrische Verfahren eingesetzt werden, bei denen die Trübung beziehungsweise die optische Dichte einer Lösung oder Suspension anhand der Lichtschwächung oder Extinktion eines direkt durch die Suspension hindurch tretenden Lichtstrahls gemessen wird.

Die Intensität des Lichtstrahls nimmt beim Durchtritt durch eine Messzelle beziehungsweise Küvette, die eine flüssige Probe enthält, ab. Die Verluste können durch Interaktionen des Lichtstrahls mit der in der Messzelle befindlichen Probe, beispielsweise durch Absorptions-, Diffraktions-, Streuungs- und/oder Reflexionseffekte beeinflusst werden. Im Allgemeinen können Diffraktions-, Beugungs- und Reflexionseffekte vernachlässigt beziehungsweise durch Referenzmessungen ausgeglichen werden, so dass hauptsächlich die Absorption zur Schwächung des Lichtstrahls beiträgt.

Photometrische Konzentrationsbestimmungen beruhen daher auf einer gesetzmäßigen Abhängigkeit der Extinktion beziehungsweise Absorption von der Konzentration der gelösten Stoffe und der Schichtdicke der Messzelle bei einer bestimmten Wellenlänge des eingestrahlten Lichts. Diesen Zusammenhang beschreibt das Lambert-Beersche Gesetz:

$$E(\lambda) = -\log(I/I_0) = \varepsilon(\lambda) \cdot c \cdot d$$

wobei $E(\lambda)$ die von der Wellenlänge $\lambda$ des Lichtstrahls abhängige Extinktion, I die Lichtintensität nach Durchtritt durch die Probe, $I_0$ die Lichtintensität vor Durchtritt durch die Probe, $\varepsilon(\lambda)$ der wellenlängenabhängige molare Extinktionskoeffizient eines durchstrahlten Stoffes, c die molare Konzentration des durchstrahlten Stoffes und d die durch den Lichtstrahl durchstrahlte Schichtdicke, beispielsweise der Messzelle ist.

Anhand der Extinktion $E(\lambda)$ einer Probe lässt sich die Konzentration einer Substanz in einer Lösung ermitteln. Dazu ist es erforderlich, dass zuvor die Extinktion mindestens einer Standardlösung bekannter Konzentration bestimmt wurde. Da sich die Extinktion proportional zur Konzentration verhält, kann mittels Kalibration durch Extinktionsmessungen mehrerer Standardlösungen bekannter Konzentrationen die Konzentration einer gelösten Substanz in einer unbekannten Probe ermittelt werden.

Die Extinktion einer Probe hängt jedoch nicht nur von der Konzentration der zu bestimmenden Substanz selbst ab, sondern auch von der Art der Probenmatrix. Die Extinktionen verschiedener Substanzen verhalten sich in einem Gemisch additiv, sofern die Substanzen nicht untereinander wechselwirken. Körperflüssigkeiten, wie beispielsweise Blutplasma oder Blutserum sind jeweils komplexe Gemische und enthalten neben dem zu bestimmenden Analyten eine Vielzahl weiterer Substanzen, die die Gesamtabsorption der Probe beeinflussen.

Körperflüssigkeitsproben können jedoch in Einzelfällen abnormal hohe Konzentrationen einer oder mehrerer intrinsischer, also körpereigener Substanzen enthalten, die sich bei Überschreitung einer tolerablen Konzentration in photometrischen Detektionsverfahren als störend erweisen und sich zu einem systematischen Fehler auswirken können.

Probleme bereiten bekanntermaßen hämolytische, ikterische und/oder lipämische Serum- oder Plasmaproben, die über abnormal hohe Hämoglobin-, Bilirubin- und/oder LipidKonzentrationen verfügen. Abnormal hohe Konzentrationen dieser interferierenden Substanzen können durch einen pathologischen Zustand des Patienten oder aber durch eine unsachgemäße Probengewinnung oder -lagerung verursacht werden. Werden solche Proben einem photometrischen Verfahren unterworfen, das der Bestimmung eines analytischen, diagnostisch relevanten Parameters dient, besteht die Gefahr einer Fehlbestimmung, die gegebenenfalls eine Fehldiagnose und schlimmstenfalls eine Fehlbehandlung des Patienten zur Folge haben kann. Die präanalytische Identifikation hämolytischer, ikterischer sowie lipämischer Proben ist also zur Vermeidung von fehlerhaften Analyseergebnissen von besonderer Wichtigkeit.

Es besteht daher ein Bedarf an Verfahren zur Ermittlung der spektrometrischen Auswirkungen störender Substanzen in Körperflüssigkeitsproben bzw. zur Identifizierung von Körperflüssigkeitsproben, die erhöhte Konzentrationen einer oder mehrerer Störsubstanzen enthalten.

In EP-A1-1059522, US 4,263,512, US 2009/0009750 A1 und US 2010/0174491 A1 sind verschiedene Verfahren zur Bestimmung von Bilirubin, Hämoglobin und Lipiden in Plasma- oder Serumproben beschrieben. In der EP-A1-1059522 wird beispielsweise die nach Abzug der Extinktion durch Hämoglobin und Bilirubin verbleibende Extinktion, die insbesondere auch die durch Lipide verursachte Extinktion enthält, lokal linear approximiert.

Auch das letztgenannte Verfahren hat jedoch den Nachteil, dass gerade eine vergleichsweise hohe Lipidkonzentration die Bestimmung von Bilirubin und Hämoglobin in derselben Probe beeinflussen und somit die Messwerte verfälschen kann.

In der WO 2013/010970 A1 und der EP-A2-3051272 sind bereits Verfahren beschrieben, die auch in Gegenwart hoher Lipidkonzentrationen eine genaue Bestimmung von Bilirubin und Hämoglobin und auch eine Bestimmung der Lipidkonzentration in einer Probe ermöglichen. Diese Verfahren umfassen im Wesentlichen die Messung der Extinktion der Probe bei verschiedenen Wellenlängen, das Berechnen einer potenzfunktionellen Näherungskurve für die Extinktion der Lipide, die Subtraktion des Hämoglobin- und Bilirubin-Anteils von den Extinktionen bis eine Lipid-Kurve übrig bleibt und schließlich die Ermittlung theoretischer Extinktionswerte unter Heranziehung von substanzspezifischen Extinktionskoeffizienten.

Problematisch ist jedoch, dass bereits bestehende Analysegeräte häufig nur über eine begrenzte Anzahl von Lichtquellen mit vorgegebenen Wellenlängen verfügen. Da die aus dem Stand der Technik bekannten Verfahren zur Bestimmung der Störsubstanzen eine bestimmte Kombination von Messwellenlängen erfordern, kann nicht jedes der bekannten Verfahren ohne Weiteres auf jedem bestehenden Analysegerät implementiert werden. Der vorliegenden Erfindung liegt daher die Aufgabe zugrunde, das Verfahren gemäß EP-A2-3051272 so zu modifizieren, dass es eine genauere Bestimmung der Lipid-, der Hämoglobin- und der Bilirubin-Konzentration in Körperflüssigkeitsproben ermöglicht, wenn andere als die in EP-A2-3051272 genannten Wellenlängen ($\lambda$1: 600-660 nm; $\lambda$2: 400-480 nm, $\lambda$3: 400-440 nm; $\lambda$4: 350-370 nm) zur Verfügung stehen, insbesondere wenn folgende Kombination von Wellenlängen zur Verfügung steht: eine Wellenlänge, bei der die nicht durch Lipide verursachte Extinktion vernachlässigbar ist (wie z.B. 660 nm); zwei Wellenlängen, bei denen die nicht durch Bilirubin und Hämoglobin und Lipide verursachte Extinktion vernachlässigbar ist (wie z.B. 340 nm und 405 nm); und eine Wellenlänge, bei der Hämoglobin ein Extinktionsmaximum aufweist (wie z.B. 575 nm).

Diese Aufgabe wird unter anderem dadurch gelöst, dass anstelle der Berechnung einer potenzfunktionellen Näherungskurve für die Extinktion der Lipide eine exponentielle Näherungskurve für die Extinktion der Lipide berechnet wird und anstelle der Extinktionskoeffizienten der Störsubstanzen vorbestimmte Proportionalitätsfaktoren zur Ermittlung der theoretischen Extinktionswerte herangezogen werden.

Gegenstand der vorliegenden Erfindung ist demnach ein Verfahren zur quantitativen Bestimmung von Lipiden, Hämoglobin und Bilirubin in einer Körperflüssigkeitsprobe mit den Schritten:

a) Durchstrahlen der Körperflüssigkeitsprobe mit Licht bei einer Vielzahl von Wellenlängen $\lambda$;

b) Erfassen eines ersten Messwertes A1 bei einer ersten Wellenlänge $\lambda$1, bei der die nicht durch Lipide verursachte Extinktion vernachlässigbar ist;

c) Erfassen eines zweiten Messwertes A2 bei einer zweiten Wellenlänge $\lambda$2, bei der die nicht durch Bilirubin und Hämoglobin und Lipide verursachte Extinktion vernachlässigbar ist;

d) Erfassen eines dritten Messwertes A3 bei einer dritten Wellenlänge $\lambda$3, bei der Hämoglobin ein Extinktionsmaximum aufweist;

e) Erfassen eines vierten Messwertes A4 bei einer vierten Wellenlänge $\lambda$4, bei der die nicht durch Bilirubin und Hämoglobin und Lipide verursachte Extinktion vernachlässigbar ist;

f) Bestimmen eines Parameters q mit der Formel:

$$q = \frac{\ln A1 - \ln A2}{\lambda 1 - \lambda 2}$$

und eines Parameters p mit der Formel:

$$p = \frac{A2}{e^{\lambda 2 \cdot q}}$$

g) Berechnen einer exponentiellen Näherungskurve ($L_0$) der Form:

$$E = f(\lambda) = p \cdot e^{\lambda \cdot q}$$

für die Extinktion E der Lipide;

h) Bestimmen eines ersten theoretischen Extinktionswertes $E_{H1}$ für Hämoglobin, der der Differenz zwischen dem dritten Messwert A3 und dem Wert der Näherungskurve ($L_0$) bei der dritten Wellenlänge $\lambda 3$ entspricht;

i) Prüfen, ob der erste theoretische Extinktionswert $E_{H1}$ für Hämoglobin größer 0 ist und wenn er größer 0 ist, Bestimmen eines zweiten theoretischen Extinktionswertes $E_{H2}$ für Hämoglobin durch Multiplikation des ersten theoretischen Extinktionswertes $E_{H1}$ für Hämoglobin mit einem ersten vorbestimmten Proportionalitätsfaktor, der das Verhältnis zwischen Extinktionswerten für Hämoglobin bei der dritten Wellenlänge $\lambda 3$ und Extinktionswerten für Hämoglobin bei der vierten Wellenlänge $\lambda 4$ widerspiegelt;

j) Bestimmen eines ersten theoretischen Extinktionswertes $E_{B1}$ für Bilirubin, der der Differenz zwischen dem vierten Messwert A4 und der Summe des Wertes der Näherungskurve ($L_0$) bei der vierten Wellenlänge $\lambda 4$ und des zweiten theoretischen Extinktionswertes $E_{H2}$ für Hämoglobin entspricht;

k) Prüfen, ob der erste theoretische Extinktionswert $E_{B1}$ für Bilirubin größer 0 ist; und

l) wenn die Prüfung in Schritt i) ergibt, dass der erste theoretische Extinktionswert $E_{H1}$ für Hämoglobin größer 0 ist, Zuordnen des ersten theoretischen Extinktionswertes $E_{H1}$ für Hämoglobin zu einem von mehreren vorab festgelegten Hämoglobin-Konzentrationsleveln, und

m) wenn die Prüfung in Schritt k) ergibt, dass der erste theoretische Extinktionswert $E_{B1}$ für Bilirubin größer 0 ist, Zuordnen des ersten theoretischen Extinktionswertes $E_{B1}$ für Bilirubin zu einem von mehreren vorab festgelegten Bilirubin-Konzentrationsleveln, und

n) Zuordnen des ersten Messwertes A1 zu einem von mehreren vorab festgelegten Lipid-Konzentrationsleveln.

In einer Ausführungsform des erfindungsgemäßen Verfahrens liegt die erste Wellenlänge $\lambda 1$, bei der die nicht durch Lipide verursachte Extinktion vernachlässigbar ist, zwischen 600 nm und 660 nm; vorzugsweise beträgt sie 660 nm.

In einer weiteren Ausführungsform des erfindungsgemäßen Verfahrens liegt die zweite Wellenlänge $\lambda 2$, bei der die nicht durch Bilirubin und Hämoglobin und Lipide verursachte Extinktion vernachlässigbar ist, zwischen 320 nm und 360 nm; vorzugsweise beträgt sie 340 nm.

In wiederum einer weiteren Ausführungsform des erfindungsgemäßen Verfahrens liegt die dritte Wellenlänge $\lambda 3$, bei der Hämoglobin ein Extinktionsmaximum aufweist, zwischen 540 nm und 580 nm; vorzugsweise beträgt sie 575 nm.

In noch einer weiteren Ausführungsform des erfindungsgemäßen Verfahrens liegt die vierte Wellenlänge $\lambda 4$, bei der die nicht durch Bilirubin und Hämoglobin und Lipide verursachte Extinktion vernachlässigbar ist, zwischen 380 nm und 440 nm; vorzugsweise beträgt sie 405 nm.

In einer besonders bevorzugten Ausführungsform beträgt die erste Wellenlänge $\lambda 1$ 660 nm und die zweite Wellenlänge $\lambda 2$ 340 nm und die dritte Wellenlänge $\lambda 3$ 575 nm und die vierte Wellenlänge $\lambda 4$ 405 nm.

"Messwerte" (A1; A2; A3 etc.) im Sinne der vorliegenden Erfindung sind Extinktionsmesswerte, welche mit photometrischen Messeinrichtungen aufgenommen werden können. Bei einem Messwert kann es sich um eine dimensionslose Größe handeln, welche ein wellenlängenabhängiges Maß für die Opazität einer Körperflüssigkeitsprobe gegenüber dem Durchgang von Lichtstrahlen im sichtbaren, infraroten und/oder ultravioletten Wellenlängenbereich angibt. Es kann gleichermaßen auch möglich sein, dass Extinktionsmesswerte in Bezug auf eine Einheitsdicke einer Messzelle oder Küvette, in der Körperflüssigkeitsproben während des Durchtritts von Lichtstrahlen zur Erfassung von Intensitätsmesswerten gehalten werden, angegeben werden. In diesem Fall können die Messwerte eine Dimension von [1/mm] aufweisen. In jedem Fall sind die angegebenen Messwerte der nachfolgenden Ausführungsformen nur beispielhafter Natur und von der Messeinrichtung, der Probenbeschaffenheit und der Probenzusammensetzung abhängig. Extinktionsmesswerte werden im Folgenden jeweils mit Absorptionswerten gleichgesetzt, obwohl es dem Fachmann klar ist, dass bei dieser Betrachtung Diffraktion, Streuung und Reflexion zwar zu den Extinktionswerten beitragen, gegenüber der Absorption jedoch im betrachteten Wellenlängenbereich im Wesentlichen vernachlässigbar sind.

"Theoretische Extinktionswerte" ($E_H$, $E_B$, $E_{HBL}$ etc.) im Sinne der vorliegenden Erfindung sind nicht tatsächlich gemessene Extinktionswerte, sondern berechnete Werte.

"Lipide" im Sinne der vorliegenden Anmeldung umfassen insbesondere im menschlichen oder tierischen Organismus vorkommende Fette bzw. Triglyceride bzw. Triacylglycerine.

Nach Aufnahme der Messwerte A1 bis A4 (Schritte a) bis e) des erfindungsgemäßen Verfahrens) und der nachfolgenden Ermittlung der theoretischen Näherungskurve für Lipid (Schritte f) und g) des erfindungsgemäßen Verfahrens) wird zunächst der Extinktionsanteil von Hämoglobin bestimmt. Dazu wird zunächst ein erster theoretischer Extinktionswertes $E_{H1}$ für Hämoglobin bestimmt, indem die Differenz zwischen dem dritten Messwert A3 und dem Wert der Näherungskurve ($L_0$) bei der dritten Wellenlänge $\lambda 3$ entspricht, und es wird geprüft, ob der erste theoretische Extinktionswert $E_{H1}$ für Hämoglobin größer 0 ist. Ergibt die Prüfung, dass er kleiner oder gleich 0 ist, wird das Fehlen von Hämoglobin festgestellt. Für nachfolgende Berechnungen wird ein theoretischer Extinktionswert für Hämoglobin von 0 verwendet. Ergibt die Prüfung hingegen, dass er größer 0 ist, wird dieser erste theoretische Extinktionswert $E_{H1}$ für Hämoglobin einem von mehreren vorab festgelegten Hämoglobin-Konzentrationsleveln zugeordnet.

Für die Bestimmung des Extinktionsanteils von Bilirubin wird zunächst ein zweiter theoretischer Extinktionswert $E_{H2}$ für Hämoglobin bestimmt, indem der erste theoretische Extinktionswert $E_{H1}$ für Hämoglobin mit einem ersten vorbestimmten Proportionalitätsfaktor multipliziert wird.

Der erste Proportionalitätsfaktor spiegelt das Verhältnis zwischen Extinktionswerten für Hämoglobin bei der dritten Wellenlänge $\lambda 3$ und Extinktionswerten für Hämoglobin bei der vierten Wellenlänge $\lambda 4$ wider und ist beispielsweise dadurch vorbestimmbar, dass in einem Vorversuch die Extinktion einer Vielzahl von Proben mit unterschiedlicher Hämoglobin-Konzentration (z.B. von 20 bis 400 mg/dL) bei der dritten Wellenlänge $\lambda 3$ und bei der vierten Wellenlänge $\lambda 4$ gemessen wird und der Mittelwert der Quotienten aus dem Messwert bei der vierten Wellenlänge $\lambda 4$ und dem Messwert bei der dritten Wellenlänge $\lambda 3$ bestimmt wird.

Ein erster theoretischer Extinktionswert $E_{B1}$ für Bilirubin wird dann bestimmt, indem die Differenz zwischen dem vierten Messwert A4 und der Summe des Wertes der Näherungskurve ($L_0$) bei der vierten Wellenlänge $\lambda 4$ und des zweiten theoretischen Extinktionswertes $E_{H2}$ für Hämoglobin bestimmt wird. Es wird geprüft, ob der erste theoretische Extinktionswert $E_{B1}$ für Bilirubin größer 0 ist. Ergibt die Prüfung, dass er kleiner oder gleich 0 ist, wird das Fehlen von Bilirubin festgestellt. Ergibt die Prüfung hingegen, dass er größer 0 ist, wird dieser erste theoretische Extinktionswert $E_{B1}$ für Bilirubin einem von mehreren vorab festgelegten Bilirubin-Konzentrationsleveln zugeordnet.

Außerdem wird der erste Messwert A1 einem von mehreren vorab festgelegten Lipid-Konzentrationsleveln zugeordnet.

Unter einem "vorab festgelegten Konzentrationslevel" (für Hämoglobin, Bilirubin oder Lipide) ist ein Teilbereich des Konzentrationsbereichs der Störsubstanz zu verstehen, der für die präanalytische Identifikation hämolytischer, ikterischer sowie lipämischer Proben relevant ist. Typischerweise wird für Hämoglobin der Konzentrationsbereich von 0 bis 1.000 mg/dl, für Bilirubin der Konzentrationsbereich von 0 bis 60 mg/dl und für Lipide der Konzentrationsbereich von 0 bis 2.000 mg/dl als relevant angesehen. Typischerweise wird der relevante Konzentrationsbereich in mindestens 5 Teilbereiche unterschiedlicher Konzentrationslevel unterteilt, denen Index-Werte (1, 2, 3, u.s.w.) zugeordnet werden. Je höher die Konzentration der Störsubstanz desto höher der Index-Wert. Diese sogenannten "HIL-Indices" sind semiquantitative Marker, die die präanalytische Identifikation hämolytischer, ikterischer sowie lipämischer Proben ermöglichen.

Die Konzentrationslevel für Hämoglobin, Bilirubin und Lipide werden typischerweise vom Hersteller des Analysesystems definiert, und es wird dann ermittelt, welche mit dem erfindungsgemäßen Verfahren bestimmten Messwerte bzw. theoretisch ermittelten Extinktionswerte, welchem Konzentrationslevel entsprechen. Dies erfolgt typischerweise, indem eine Vielzahl von Proben mit unterschiedlicher Hämoglobin-Konzentration (z.B. von 0 bis 1.000 mg/dl Hämoglobin) und eine weitere Vielzahl von Proben mit unterschiedlicher Bilirubin-Konzentration (z.B. von 0 bis 60 mg/dl Bilirubin) und eine weitere Vielzahl von Proben mit unterschiedlicher Lipid-Konzentration (z.B. von 0 bis 2.000 mg/dl Intralipid) mit dem erfindungsgemäßen Verfahren analysiert werden und die Messwerte bzw. theoretisch ermittelten Extinktionswerte den definierten Konzentrationsleveln zugeordnet werden.

In einer Ausführungsform des erfindungsgemäßen Verfahrens wird nach der Berechnung der Näherungskurve $L_0$ für die Extinktion E der Lipide in Schritt g) eine korrigierte Näherungskurve $L_k$ für die Extinktion E der Lipide erstellt, indem die Näherungskurve $L_0$ mittels eines iterativen Verfahrens korrigiert wird. Dies bewirkt eine präzisere Bestimmung der Störsubstanzen, insbesondere in Proben die einen hohen Lipidgehalt aufweisen.

Vorzugsweise wird eine korrigierte Näherungskurve $L_k$ für die Extinktion E der Lipide nur dann erstellt, wenn festgestellt wird, dass der Quotient aus der Differenz zwischen dem vierten Messwert A4 und dem Wert der Näherungskurve ($L_0$) bei der vierten Wellenlänge $\lambda 4$ und aus dem vierten Messwert A4 größer 0,2 ist.

Vorzugsweise werden für die Erstellung der korrigierten Näherungskurve $L_k$ ein dritter theoretischer Extinktionswert $E_{H3}$ für Hämoglobin, ein zweiter theoretischer Extinktionswert $E_{B2}$ für Bilirubin und ein erster theoretischer Extinktionswert $E_{L1korr}$ für Lipide bestimmt.

Der dritte theoretische Extinktionswert $E_{H3}$ für Hämoglobin wird ermittelt durch Multiplikation des ersten theoretischen Extinktionswertes $E_{H1}$ für Hämoglobin mit einem zweiten vorbestimmten Proportionalitätsfaktor.

Der zweite Proportionalitätsfaktor spiegelt das Verhältnis zwischen Extinktionswerten für Hämoglobin bei der dritten Wellenlänge $\lambda 3$ und Extinktionswerten für Hämoglobin bei der zweiten Wellenlänge $\lambda 2$ wider und ist beispielsweise dadurch vorbestimmbar, dass in einem Vorversuch die Extinktion einer Vielzahl von Proben mit unterschiedlicher Hämoglobin-Konzentration (z.B. von 20 bis 400 mg/dl) bei der dritten Wellenlänge $\lambda 3$ und bei der zweiten Wellenlänge $\lambda 2$ gemessen wird und der Mittelwert der Quotienten aus dem Messwert bei der zweiten Wellenlänge $\lambda 2$ und dem Messwert bei der dritten Wellenlänge $\lambda 3$ bestimmt wird.

Der zweite theoretische Extinktionswert $E_{B2}$ für Bilirubin wird bestimmt durch Multiplikation des ersten theoretischen Extinktionswertes $E_{B1}$ für Bilirubin mit einem dritten vorbestimmten Proportionalitätsfaktor.

Der dritte Proportionalitätsfaktor spiegelt das Verhältnis zwischen Extinktionswerten für Bilirubin bei der zweiten Wellenlänge λ2 und Extinktionswerten für Bilirubin bei der vierten Wellenlänge λ4 wider und ist beispielsweise dadurch vorbestimmbar, dass in einem Vorversuch die Extinktion einer Vielzahl von Proben mit unterschiedlicher Bilirubin-Konzentration (z.B. von 1 bis 60 mg/dl) bei der zweiten Wellenlänge λ2 und bei der vierten Wellenlänge λ4 gemessen wird und der Mittelwert der Quotienten aus dem Messwert bei der zweiten Wellenlänge λ2 und dem Messwert bei der vierten Wellenlänge λ4 bestimmt wird.

Der erste theoretische Extinktionswert $E_{L1korr}$ für Lipide wird bestimmt, indem die Differenz zwischen dem zweiten Messwert A2 und der Summe des dritten theoretischen Extinktionswertes $E_{H3}$ für Hämoglobin und des zweiten theoretischen Extinktionswertes $E_{B2}$ für Bilirubin ermittelt wird.

Vorteilhafterweise erfolgt das Durchstrahlen der Körperflüssigkeitsprobe mit Licht bei einer Vielzahl von Wellenlängen mit Hilfe von Laser- oder Leuchtdioden oder mit Hilfe einer Lichtquelle mit verschiedenen optischen Filtern und das Erfassen der Vielzahl von Messwerten (A1; A2; A3; A4) mit Hilfe eines Photodetektors, z.B. mit einem CCD-Sensor, einem CMOS-Sensor, Photosensoren oder ähnlichen Einrichtungen, welche dazu geeignet sind, die Intensität eines Lichtstrahls wellenlängenabhängig zu erfassen. "Körperflüssigkeitsproben" im Sinne der vorliegenden Erfindung können alle Proben biologischen Ursprungs sein, welche flüssige Konsistenz aufweisen und eine Vielzahl von biologisch aktiven Substanzen in verschiedenen Konzentrationen aufweisen. Beispielsweise können Körperflüssigkeitsproben Blutserum, Blutplasma, Blut, Urin, Lymphflüssigkeit, Gallenflüssigkeit oder ähnliche Flüssigkeiten aufweisen.

Ein weiterer Gegenstand der vorliegenden Erfindung ist ein automatisches Analysegerät mit einer Messeinrichtung, wobei die Messeinrichtung mindestens eine Lichtquelle und mehrere optische Filter oder mehrere Lichtquellen, vorzugsweise mehrere Leucht- oder Laserdioden und mindestens einen Photodetektor umfasst, wobei die Messeinrichtung dazu ausgelegt ist, die Verfahrensschritte a) bis e) des erfindungsgemäßen Verfahrens auszuführen und welches ferner eine Berechnungseinrichtung, z. B. einen Prozessor, umfasst, welche dazu ausgelegt ist, die übrigen Verfahrensschritte f) bis n) zur quantitativen Bestimmung von Lipiden, Hämoglobin und Bilirubin des weiter oben beschriebenen, erfindungsgemäßen Verfahrens auszuführen.

In einer Ausführungsform umfasst die Messeinrichtung des automatischen Analysegeräts mindestens eine Lichtquelle und mehrere optische Filter zur Generierung von Licht unterschiedlicher Wellenlängen. In einer anderen Ausführungsform umfasst die Messeinrichtung mehrere Lichtquellen, vorzugsweise mehrere Leucht- oder Laserdioden.

In einer besonders bevorzugten Ausführungsform umfasst die Messeinrichtung des automatischen Analysegeräts mindestens vier Lichtquellen, wobei die erste Lichtquelle Licht einer Wellenlänge im Bereich zwischen 600 nm und 660 nm emittiert, und die zweite Lichtquelle Licht einer Wellenlänge im Bereich zwischen 320 nm und 360 nm emittiert, und die dritte Lichtquelle Licht einer Wellenlänge im Bereich zwischen 540 nm und 580 nm emittiert, und die vierte Lichtquelle Licht einer Wellenlänge im Bereich zwischen 380 nm und 440 nm emittiert.

In einer besonders bevorzugten Ausführungsform umfasst die Messeinrichtung des automatischen Analysegeräts mindestens vier Lichtquellen, wobei die erste Lichtquelle Licht einer Wellenlänge von 660 nm emittiert, und die zweite Lichtquelle Licht einer Wellenlänge von 340 nm emittiert, und die dritte Lichtquelle Licht einer Wellenlänge von 575 nm emittiert, und die vierte Lichtquelle Licht einer Wellenlänge von 405 nm emittiert.

Vorteilhafterweise umfasst die Messeinrichtung außerdem mindestens einen Photodetektor, z.B. einen CCD-Sensor, einen CMOS-Sensor, Photosensoren oder ähnliche Einrichtungen, welche dazu geeignet sind, die Intensität eines Lichtstrahls wellenlängenabhängig zu erfassen.

Verschiedene Ausführungsbeispiele und Ausgestaltungen der vorliegenden Erfindung werden nun in Bezug auf die beiliegenden Zeichnungen genauer beschrieben.

FIGURENBESCHREIBUNG

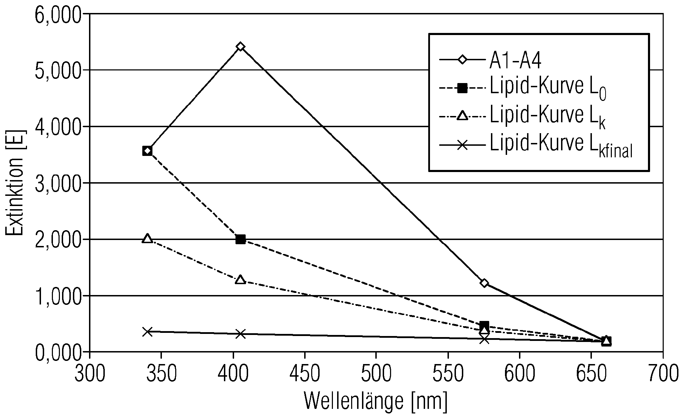

FIG. 1     zeigt den tatsächlich gemessenen Extinktionsverlauf einer Probe (Nr. 3417; A1-A4; Rauten) sowie die berechneten Lipid-Kurven $L_0$ (Vierecke), $L_k$ (Dreiecke) und $L_{kfinal}$ (Kreuze) .

**BEISPIEL**

**a) Wellenlängen**

Das erfindungsgemäße Verfahren wurde in einem automatischen Analysegerät umfassend eine photometrische Anordnung mit vier Laserdioden durchgeführt. Humane Plasmaproben wurden in einer Schichtdicke von d = 1 mm mit Licht folgender Wellenlängen durchstrahlt:

660 nm     erste Wellenlänge λ1, bei der die nicht durch Lipide verursachte Extinktion vernachlässigbar ist;

(fortgesetzt)

| 340 nm | zweite Wellenlänge $\lambda 2$, bei der die nicht durch Bilirubin und Hämoglobin und Lipide verursachte Extinktion vernachlässigbar ist; |
| 575 nm | dritte Wellenlänge $\lambda 3$, bei der Hämoglobin ein Extinktionsmaximum aufweist; |
| 405 nm | vierte Wellenlänge $\lambda 4$, bei der die nicht durch Bilirubin und Hämoglobin und Lipide verursachte Extinktion vernachlässigbar ist. |

Die o. g. vier Messwerte ($A1 = A_{660}$, $A2 = A_{340}$, $A3 = A_{575}$ und $A4 = A_{405}$) wurden aufgezeichnet.

**b) Vorbestimmung der Proportionalitätsfaktoren**

**Erster und zweiter Proportionalitätsfaktor**

Der erste Proportionalitätsfaktor ($F_{HB405}$) spiegelt das Verhältnis zwischen Extinktionswerten für Hämoglobin bei der dritten Wellenlänge $\lambda 3 = 575$ nm und Extinktionswerten für Hämoglobin bei der vierten Wellenlänge $\lambda 4 = 405$ nm wider. Der zweite Proportionalitätsfaktor ($F_{HB340}$) spiegelt das Verhältnis zwischen Extinktionswerten für Hämoglobin bei der dritten Wellenlänge $\lambda 3 = 575$ nm und Extinktionswerten für Hämoglobin bei der zweiten Wellenlänge $\lambda 2 = 340$ nm wider. In einem Vorversuch wurde die Extinktion von 16 Proben mit unterschiedlicher Hämoglobin-Konzentration (von 20 bis 400 mg/dl) bei 575 nm, bei 405 nm und bei 340 nm gemessen, und der Mittelwert der Quotienten aus den Messwerten bei 405 nm und den Messwerten bei 575 nm (erster Proportionalitätsfaktor) bzw. der Mittelwert der Quotienten aus den Messwerten bei 340 nm und den Messwerten bei 575 nm (zweiter Proportionalitätsfaktor) wurde bestimmt. Die in den verschiedenen Proben ermittelten Messwerte und die daraus ermittelten Quotienten sind in Tabelle 1 gezeigt. Für den ersten Proportionalitätsfaktor ($F_{HB405}$) wurde so ein Wert von 6,3 ermittelt. Für den zweiten Proportionalitätsfaktor ($F_{HB340}$) wurde so ein Wert von 1,9 ermittelt.

**Tabelle 1**

| Probe Hämoglobin [mg/dL] | $A_{340}$ ($\lambda 2$=340 nm) | $A_{575}$ ($\lambda 3$=575 nm) | $A_{405}$ ($\lambda 4$=405 nm) | $A_{405}$/ $A_{575}$ | $A_{340}$/ $A_{575}$ |
|---|---|---|---|---|---|
| 20 | 0,039 | 0,021 | 0,130 | 6,2 | 1,9 |
| 40 | 0,075 | 0,040 | 0,252 | 6,3 | 1,9 |
| 60 | 0,113 | 0,060 | 0,377 | 6,3 | 1,9 |
| 80 | 0,156 | 0,082 | 0,519 | 6,3 | 1,9 |
| 100 | 0,187 | 0,099 | 0, 617 | 6,2 | 1,9 |
| 120 | 0,226 | 0,120 | 0,753 | 6,3 | 1,9 |
| 140 | 0,263 | 0,139 | 0,877 | 6,3 | 1,9 |
| 160 | 0,303 | 0,159 | 1,007 | 6,3 | 1,9 |
| 180 | 0,327 | 0,172 | 1,087 | 6,3 | 1,9 |
| 200 | 0,381 | 0,201 | 1,261 | 6,3 | 1,9 |
| 240 | 0,442 | 0,233 | 1,462 | 6,3 | 1,9 |
| 260 | 0,458 | 0,241 | 1,518 | 6,3 | 1,9 |
| 300 | 0,575 | 0,303 | 1,903 | 6,3 | 1,9 |
| 340 | 0,653 | 0,344 | 2,164 | 6,3 | 1,9 |
| 380 | 0,709 | 0,373 | 2,339 | 6,3 | 1,9 |
| 400 | 0,734 | 0,387 | 2,442 | 6,3 | 1,9 |
| | | | **Mittelwert** | **6,3** | **1,9** |

**Dritter Proportionalitätsfaktor**

Der dritte Proportionalitätsfaktor ($F_{B340}$) spiegelt das Verhältnis zwischen Extinktionswerten für Bilirubin bei

der zweiten Wellenlänge $\lambda 2$ = 340 nm und Extinktionswerten für Bilirubin bei der vierten Wellenlänge $\lambda 4$ = 405 nm wider.

In einem Vorversuch wurde die Extinktion von 13 Proben mit unterschiedlicher Bilirubin-Konzentration (von 1,2 bis 60 mg/dl) bei 340 nm und bei 405 nm gemessen, und der Mittelwert der Quotienten aus den Messwerten bei 340 nm und den Messwerten bei 405 nm wurde bestimmt. Die in den verschiedenen Proben ermittelten Messwerte und die daraus ermittelten Quotienten sind in Tabelle 2 gezeigt. Für den dritten Proportionalitätsfaktor ($F_{B340}$) wurde so ein Wert von 0,22 ermittelt.

**Tabelle 2**

| Probe Bilirubin [mg/dL] | $A_{340}$ ($\lambda 2$=340 nm) | $A_{405}$ ($\lambda 4$=405 nm) | $A_{340}$/ $A_{575}$ |
|---|---|---|---|
| 1,2 | 0,013 | 0,055 | 0,23 |
| 2,4 | 0,024 | 0,108 | 0,22 |
| 4,8 | 0,049 | 0,225 | 0,22 |
| 6 | 0,061 | 0,280 | 0,22 |
| 8,4 | 0,086 | 0,391 | 0,22 |
| 12 | 0,113 | 0,514 | 0,22 |
| 18 | 0,176 | 0,801 | 0,22 |
| 24 | 0,236 | 1,069 | 0,22 |
| 30 | 0,300 | 1,339 | 0,22 |
| 36 | 0,361 | 1,585 | 0,23 |
| 42 | 0,418 | 1,819 | 0,23 |
| 48 | 0,461 | 1,990 | 0,23 |
| 60 | 0,587 | 2,524 | 0,23 |
| | | **Mittelwert** | **0,22** |

### c) Festlegung der Konzentrationslevel und Indexwerte

Eine humane Plasmaprobe mit einer Hämoglobinkonzentration von 120 mg/dl wurde in einer Schichtdicke von d = 5 mm mit Licht der Wellenlänge 575 nm ($\lambda 3$) durchstrahlt, und es wurde ein Extionsktionsmesswert von ca. 619 mE, gerundet 0,6 E, gemessen. Eine Extinktion von 0 bis 0,6 E entspricht also einem Konzentrationlevel von 0 bis 120 mg/dL Hämoglobin; dieser Bereich wurde als "Level 0" definiert. Da sich Hämoglobin in der Absorption proportional verhält, wurde die Leveldefinition entsprechend bis Level 5 fortgesetzt, d. h. bis 1,2 E ist dann Level 1, bis 1,8 E ist dann Level 2 und so weiter.

Für Bilirubin (Bilirubinkonzentration 4 mg/dl; Extinktionsmessung bei 405 nm) und Lipid (Intralipidkonzentration 60 mg/dl; Extinktionsmessung bei 660 nm) wurde analog verfahren.

Die so bestimmten Konzentrationslevel sind in Tabelle 3 aufgeführt.

**Tabelle 3**

| Extinktion (E) | Level | Hämoglobin [mg/dl] | Bilirubin [mg/dl] | Lipid [mg/dl] |
|---|---|---|---|---|
| 0 bis < 0,6 | **0** | 0-120 | 0-4 | 0-60 |
| 0,6 bis < 1,2 | **1** | 120-240 | 4-8 | 60-120 |
| 1,2 bis < 1,8 | **2** | 240-360 | 8-12 | 120-180 |
| 1,8 bis < 2,4 | **3** | 360-480 | 12-16 | 180-240 |
| 2,4 bis < 3,0 | **4** | 480-600 | 16-20 | 240-300 |
| > 3,0 | **5** | > 600 | > 20 | > 300 |

### d) Ausführungsbeispiele

Es wurden vier Plasmaproben mit bekannten Störfaktorkonzentrationen (Tabelle 4) in einer Schichtdicke von d = 5 mm mit Licht der Wellenlängen 660 nm ($\lambda$1), 340 nm ($\lambda$2), 575 nm ($\lambda$3) und 405 nm ($\lambda$4) durchstrahlt, und es wurden jeweils die Extionsktionsmesswerte A1-A4 (Tabelle 5) gemessen.

**Tabelle 4**

| Proben-Nr. | Hämoglobin | Bilirubin | Lipide |
|---|---|---|---|
| 3417 | 174 mg/dl | 1,1 mg/dl | 38 mg/dl |
| 5763 | 82 mg/dl | 30 mg/dl | 58 mg/dl |
| 4217 | 130 mg/dl | 26,5 mg/dl | 47 mg/dl |
| 3283 | 25 mg/dl | 3,6 mg/dl | 32 mg/l |

**Tabelle 5**

| Proben-Nr. | A1 ($\lambda$1 = 660 nm) | A2 ($\lambda$2 = 340 nm) | A3 ($\lambda$3 = 575 nm) | A4 ($\lambda$4 = 405 nm) |
|---|---|---|---|---|
| 3417 | 0,198 E | 3,575 E | 1,240 E | 5,441 E |
| 5763 | 0,535 E | 3,906 E | 0,735 E | 5,371 E |
| 4217 | 0,347 E | 3,894 E | 0,573 E | 5,306 E |
| 3283 | 0,465 E | 3,155 E | 0,722 E | 2,526 E |

### e) Proben-Nr. 3417

Die Probe mit der Proben-Nr. 3417 weist einen leicht erhöhten Hämoglobingehalt und relativ geringe Bilirubin- und Lipidkonzentrationen auf.

### Schritt 1: Bestimmung der Lipid-Kurve $L_0$

Zur Bestimmung der exponentiellen Näherungskurve für die Extinktion der Lipide (Lipid-Kurve $L_0$) werden zunächst die gemessenen Extinktionen A1 und A2 der Lipid-Kurve zugewiesen; A1 wird nicht verändert (Ankerpunkt):

$$A1 = A_{660} = E_{L660}$$
$$A2 = A_{340} = E_{L340}$$

Die theoretischen Lipid-Extinktionen bei den Wellenlängen 405 nm ($E_{L405}$) und 575 nm ($E_{L575}$) werden mit der Formel

$$E = f(\lambda) = p \cdot e^{\lambda \cdot q}$$

bestimmt.

Zunächst werden jedoch die Parameter q und p mit den folgenden Formeln bestimmt:

$$q = \frac{\ln A1 - \ln A2}{\lambda 1 - \lambda 2}$$

und

$$p = \frac{A2}{e^{\lambda 2 \cdot q}}$$

also

$$q = \frac{\ln(0,198) - \ln(3,575)}{660 - 340} = -0,0090$$

und

$$p = \frac{3,575}{e^{340 \cdot -0,0090}} = 76,25.$$

Mit diesen Parametern werden nun die theoretischen Extinktionen der Lipid-Kurve $L_0$ an den Wellenlängen $\lambda3$ (575 nm) und $\lambda4$ (405 nm) berechnet. Es kommt folgende Formel zur Anwendung:

$$E_{Lxxx} = p \cdot e^{\lambda nx \cdot q}$$

mit $n_1$ = 405 nm, $n_2$ = 575 nm
also

$$E_{L405} = 76,25 \cdot e^{405 \cdot -0,0090} = 1,992 E$$

$$E_{L575} = 76,25 \cdot e^{575 \cdot -0,0090} = 0,431 E$$

FIG. 1 zeigt den tatsächlich gemessenen Extinktionsverlauf (A1-A4; Rauten) der Proben-Nr. 3417 sowie die so berechnete Lipid-Kurve $L_0$ (Quadrate).

**Schritt 2: Bestimmen der Extinktionsanteile von Hämoglobin und Bilirubin und der Konzentrationslevel von Hämoglobin, Bilirubin und Lipid**

**Hämoglobin**

Zur Bestimmung des Extinktionsanteils von Hämoglobin wird ein erster theoretischer Extinktionswert $E_{H1}$ bestimmt, indem zunächst die Differenz zwischen dem tatsächlichen Extinktionsmesswert A3 und der Lipid-Kurve $L_0$ bei $\lambda3$ (575 nm) bestimmt wird, und wenn der so bestimmte Extinktionswert $E_{H1}$ größer 0 ist, dann ein zweiter theoretischer Extinktionswert $E_{H2}$ bestimmt wird durch Multiplikation des Extinktionswertes $E_{H1}$ mit dem ersten vorbestimmten Proportionalitätsfaktor $F_{H405}$ (= 6,3; siehe oben unter b) und Tabelle 1), der das Verhältnis zwischen Extinktionswerten für Hämoglobin bei der dritten Wellenlänge $\lambda3$ (575 nm) und Extinktionswerten für Hämoglobin bei der vierten Wellenlänge $\lambda4$ (405 nm) widerspiegelt:

$$E_{H1} = A3 - E_{L575} = 1,240\ E - 0,431\ E = 0,809\ E;$$

$$E_{H2} = F_{H405} \bullet E_{H1} = 6,3 \bullet 0,809\ E = 5,097\ E.$$

Es wird geprüft, ob $E_{H1}$ größer oder kleiner null ist.
Im Falle von $E_{H1} > 0$ wird der theoretische Extinktionswert $E_{H1}$ einem Hämoglobin-Konzentrationslevel (siehe Tabelle 3) zugeordnet.
Im vorliegenden Fall entspricht der Extinktionswert $E_{H1}$ = 0,809 E dem Hämoglobin-Konzentrationslevel 1 (siehe Tabelle 3).

**Bilirubin**

Zur Bestimmung des Extinktionsanteils von Bilirubin wird ein erster theoretischer Extinktionswert $E_{B1}$ bestimmt, indem zunächst die Differenz zwischen dem tatsächlichen Extinktionsmesswert A4 und der Summe des Wertes der Lipid-Kurve $L_0$ bei $\lambda 4$ (405 nm) und des zweiten theoretischen Extinktionswertes $E_{H2}$ bestimmt wird, und wenn der so bestimmte Extinktionswert $E_{B1}$ kleiner 0 ist, der Extinktionswert $E_{B1}$ gleich null gesetzt wird:

$$
\begin{aligned}
E_{B1} &= A4 - E_{L405} - E_{H2} \\
&= 5,441\ E - 1,992\ E - 5,097\ E \\
&= -1,648\ E \\
&= 0\ E.
\end{aligned}
$$

Die Proben-Nr. 3417 enthält demnach keine bzw. keine nennenswerte Bilirubinkonzentration.

Im vorliegenden Fall entspricht der Extinktionswert $E_{B1}$= 0 E dem Bilirubin-Konzentrationslevel 0 (siehe Tabelle 3).

**Lipid**

Der tatsächliche Extinktionswert A1 wird einem Lipid-Konzentrationslevel (siehe Tabelle 3) zugeordnet.

Im vorliegenden Fall entspricht der Extinktionswert A1 = 0,198 E dem Lipid-Konzentrationslevel 0 (siehe Tabelle 3).

**Tabelle 6: Ergebnis für Proben-Nr. 3417:**

|  | Hämoglobin | Bilirubin | Triglycerid |
|---|---|---|---|
| Tatsächliche Konzentration | 174 mg/dl | 1,1 mg/dl | 38 mg/dl |
| Erfindungsgemäß bestimmtes Level | **1** (120-240 mg/dl) | **0** (0-4 mg/dl) | **0** (0-60 mg/dl) |

Alle bestimmten Level entsprechen den in der Probe enthaltenen Störsubstanzkonzentrationen.

**Erstellung einer korrigierten Lipid-Kurve $L_k$**

Es kann zusätzlich nach der Berechnung der Näherungskurve $L_0$ für die Extinktion E der Lipide eine korrigierte Näherungskurve $L_k$ für die Extinktion E der Lipide erstellt werden, indem die Näherungskurve $L_0$ mittels eines iterativen Verfahrens korrigiert wird. Dies bewirkt eine präzisere Bestimmung der Störsubstanzen, insbesondere in Proben die einen hohen Lipidgehalt aufweisen.

Typischerweise wird eine korrigierte Näherungskurve $L_k$ für die Extinktion E der Lipide nur dann erstellt, wenn festgestellt wird, dass der Quotient (VL) aus der Differenz zwischen dem vierten Messwert A4 und dem Wert der Näherungskurve ($L_0$) bei der vierten Wellenlänge $\lambda 4$ und aus dem vierten Messwert A4 größer 0,2 ist.

Für den Fall der Proben-Nr. 3417 ist der Quotient VL größer 0,2:

$$
VL = \frac{A4 - E_{L405}}{A4}
$$

also

$$
VL = \frac{5,441\ E - 1,922\ E}{5,441\ E} = 0,63.
$$

Die Extinktion für Lipid bei der Wellenlänge $\lambda 2$ (340 nm) (entsprechend dem tatsächlichen Messwert A2) wird durch Subtraktion der Extinktionsanteile von Hämoglobin und Bilirubin bei der dieser Wellenlänge korrigiert.

Dazu werden zunächst die Extinktionsanteile von Hämoglobin und Bilirubin an der Gesamtextinktion bei $\lambda 2$

(340 nm) ermittelt.

Zur Bestimmung des Extinktionsanteils von Hämoglobin wird ein dritter theoretischer Extinktionswert $E_{H3}$ bestimmt, indem durch Multiplikation des Extinktionswertes $E_{H1}$ für Hämoglobin mit dem zweiten vorbestimmten Proportionalitätsfaktor $F_{HB340}$ (= 1,9; siehe oben unter b) und Tabelle 1), der das Verhältnis zwischen Extinktionswerten für Hämoglobin bei der dritten Wellenlänge $\lambda 3$ (575 nm) und Extinktionswerten für Hämoglobin bei der zweiten Wellenlänge $\lambda 2$ (340 nm) widerspiegelt:

$$E_{H3} = F_{HB340} \bullet E_{H1} = 1,9 \bullet 0,809\ E = 1,537\ E.$$

Zur Bestimmung des Extinktionsanteils von Bilirubin wird ein zweiter theoretischer Extinktionswert $E_{B2}$ bestimmt, indem durch Multiplikation des Extinktionswertes $E_{B1}$ für Bilirubin mit dem dritten vorbestimmten Proportionalitätsfaktor $F_{B340}$ (= 0,22; siehe oben unter b) und Tabelle 2), der das Verhältnis zwischen Extinktionswerten für Bilirubin bei der zweiten Wellenlänge $\lambda 2$ (340 nm) und Extinktionswerten für Bilirubin bei der vierten Wellenlänge $\lambda 4$ (405 nm) widerspiegelt:

$$E_{B2} = F_{B340} \bullet E_{B1} = 0,22 \bullet 0\ E = 0\ E.$$

Nun wird kann der theoretische Extinktionswert $E_{L1korr}$ für Lipide bei 340 nm bestimmt werden, indem die Differenz zwischen dem zweiten Messwert A2 und der Summe des dritten theoretischen Extinktionswertes $E_{H3}$ für Hämoglobin und des zweiten theoretischen Extinktionswertes $E_{B2}$ für Bilirubin gebildet wird:

$$\begin{aligned} E_{L1korr} \quad &= A2 - E_{H3} - E_{B2} \\ &= 3,575\ E - 1,537\ E - 0\ E \\ &= 2,038\ E. \end{aligned}$$

Mit diesem berechneten korrigierten Extinktionswert werden nun neue Parameter q und p für die Exponentialfunktion berechnet:

$$q = \frac{\ln A1 - \ln E_{L1korr}}{\lambda 1 - \lambda 2}$$

und

$$p = \frac{E_{L1korr}}{e^{\lambda 2 \cdot q}}$$

also

$$q = \frac{\ln(0,198) - \ln(2,038)}{660 - 340} = -0,0073$$

und

$$p = \frac{2,038}{e^{340 \cdot -0,0073}} = 24,38.$$

Mit diesen Parametern werden nun die theoretischen Extinktionen der korrigierten Lipid-Kurve $L_k$ an den Wellenlängen $\lambda 3$ (575 nm) und $\lambda 4$ (405 nm) berechnet. Es kommt folgende Formel zur Anwendung:

$$E_{L405korr} = 24,38 \bullet e^{405 \cdot -0,0073} = 1,268\ E$$

$$E_{L575korr} = 24,38 \cdot e^{575 \cdot -0,0073} = 0,367 \ E$$

Wie in FIG. 1 gezeigt, "wandert" die korrigierte Lipid-Kurve $L_k$ (Dreiecke) nach unten. Es ist das Ziel, die Kurve so weit nach unten zu schieben, bis die Differenz-Extinktion zwischen dem tatsächlichen Messwert A2 und dem theoretisch berechneten Extinktionswert für Lipide bei 340 nm der Summe der von Hämoglobin und Bilirubin verursachten Extinktionsanteile entspricht.

Dazu werden die Extinktionsanteile von Hämoglobin und Bilirubin wie bereits oben beschrieben entsprechend bestimmt und von A2 abgezogen. Das gesamte Verfahren wird iterativ mit jeweils neu zu berechnenden Parametern q und p der Exponentialfunktion erneut durchgeführt, solange bis eines der folgenden Abbruchkriterien: $E_{L1korr} < 0$ oder $q > 0$ oder $E_{L575} < A_4$ oder $E_{B1}(n) < E_{B1} (n-1)$ erreicht ist. In FIG. 1 ist die iterativ korrigierte Lipid-Kurve $L_{kfinal}$ (Kreuze) gezeigt, bei der das Abbruchkriterium $E_{L1korr} < 0$ erreicht ist. Die korrigierten Extinktionswerte dieser Kurve werden dann für die Berechnung der theoretischen Extinktionswerte für Hämoglobin und Bilirubin verwendet, die dann einem Konzentrationslevel (siehe Tabelle 3) zuzuordnen sind.

Mit der iterativ korrigierten Lipid-Kurve $L_{kfinal}$ wurden folgende theoretischen Extinktionswerte für Hämoglobin und Bilirubin bestimmt:

$E_H$ = 1,003 E
$E_B$ = 0 E

Der Extinktionswert $E_H$= 1,003 E entspricht dem Hämoglobin-Konzentrationslevel 1 (siehe Tabelle 3).

Der Extinktionswert $E_B$= 0 E entspricht dem Bilirubin-Konzentrationslevel 0 (siehe Tabelle 3).

Auch in diesem Fall entspricht der tatsächliche Extinktionswert A1 = 0,198 E dem Lipid-Konzentrationslevel 0 (siehe Tabelle 3).

**f) Proben-Nr. 5763, 4217 und 3283**

Die übrigen Proben wurden in analoger Weise gemessen, ausgewertet, und es wurden die Konzentrationslevel bestimmt.

Wie aus Tabelle 7 hervorgeht, wurden die anhand der tatsächlichen Konzentrationen erwarteten Konzentrationslevel für alle drei Störsubstanzen fast vollständig korrekt bestimmt. Lediglich bei Proben-Nr. 4217 wurden die Level für Hämoglobin und Bilirubin jeweils 1 Level zu niedrig bestimmt. Abweichungen von +/- 20 % sind bei optischen Verfahren jedoch akzeptabel. Erfahrungsgemäß kann in Proben mit hoher Bilirubin-Konzentration auch die Extinktion bei 575 nm ($\lambda$3), also bei der Wellenlänge, bei der Hämoglobin ein Extinktionsmaximum aufweist, vermindert sein, so dass es dazu kommen kann, dass zu wenig Hämoglobin bestimmt wird. Klinisch relevant ist dies aber nicht, da bereits durch die hohe Bilirubin-Konzentration klar ist, dass die Probe Störgrößen enthält.

**Tabelle 7: Ergebnis für Proben-Nr. 5763, 4217 und 3283**

| | | Hämoglobin | Bilirubin | Triglycerid |
|---|---|---|---|---|
| 5763 | Tatsächliche Konzentration | 82 mg/dl | 30 mg/dl | 58 mg/dl |
| | Erfindungs-gemäß bestimmtes Level | 0 (0-120 mg/dl) | 5 (>20 mg/dl) | 0 (0-60 mg/dl) |
| 4217 | Tatsächliche Konzentration | 130 mg/dl | 26,5 mg/dl | 47 mg/dl |
| | Erfindungs-gemäß bestimmtes Level | 0 (0-120 mg/dl) | 4 (16-20 mg/dl) | 0 (0-60 mg/dl) |
| 3283 | Tatsächliche Konzentration | 25 mg/dl | 3,6 mg/dl | 32 mg/l |
| | Erfindungs-gemäß bestimmtes Level | 0 (0-120 mg/dl) | 0 (0-4 mg/dl) | 0 (0-60 mg/dl) |

**Patentansprüche**

1. Verfahren zur quantitativen Bestimmung von Lipiden, Hämoglobin und Bilirubin in einer Körperflüssigkeitsprobe mit den Schritten:

a) Durchstrahlen der Körperflüssigkeitsprobe mit Licht bei einer Vielzahl von Wellenlängen $\lambda$;
b) Erfassen eines ersten Messwertes A1 bei einer ersten Wellenlänge $\lambda$1, bei der die nicht durch Lipide verursachte Extinktion vernachlässigbar ist;
c) Erfassen eines zweiten Messwertes A2 bei einer zweiten Wellenlänge $\lambda$2, bei der die nicht durch Bilirubin und Hämoglobin und Lipide verursachte Extinktion vernachlässigbar ist;
d) Erfassen eines dritten Messwertes A3 bei einer dritten Wellenlänge $\lambda$3, bei der Hämoglobin ein Extinktionsmaximum aufweist;
e) Erfassen eines vierten Messwertes A4 bei einer vierten Wellenlänge $\lambda$4, bei der die nicht durch Bilirubin und Hämoglobin und Lipide verursachte Extinktion vernachlässigbar ist;
f) Bestimmen eines Parameters q mit der Formel:

$$q = \frac{\ln A1 - \ln A2}{\lambda 1 - \lambda 2}$$

und eines Parameters p mit der Formel:

$$p = \frac{A2}{e^{\lambda 2 \cdot q}}$$

g) Berechnen einer exponentiellen Näherungskurve ($L_0$) der Form:

$$E = f(\lambda) = p \cdot e^{\lambda \cdot q}$$

für die Extinktion E der Lipide;

h) Bestimmen eines ersten theoretischen Extinktionswertes $E_{H1}$ für Hämoglobin, der der Differenz zwischen dem dritten Messwert A3 und dem Wert der Näherungskurve ($L_0$) bei der dritten Wellenlänge $\lambda3$ entspricht;

i) Prüfen, ob der erste theoretische Extinktionswert $E_{H1}$ für Hämoglobin größer 0 ist und wenn er größer 0 ist, Bestimmen eines zweiten theoretischen Extinktionswertes $E_{H2}$ für Hämoglobin durch Multiplikation des ersten theoretischen Extinktionswertes $E_{H1}$ für Hämoglobin mit einem ersten vorbestimmten Proportionalitätsfaktor, der das Verhältnis zwischen Extinktionswerten für Hämoglobin bei der dritten Wellenlänge $\lambda3$ und Extinktionswerten für Hämoglobin bei der vierten Wellenlänge $\lambda4$ widerspiegelt;

j) Bestimmen eines ersten theoretischen Extinktionswertes $E_{B1}$ für Bilirubin, der der Differenz zwischen dem vierten Messwert A4 und der Summe des Wertes der Näherungskurve ($L_0$) bei der vierten Wellenlänge $\lambda4$ und des zweiten theoretischen Extinktionswertes $E_{H2}$ für Hämoglobin entspricht;

k) Prüfen, ob der erste theoretische Extinktionswert $E_{B1}$ für Bilirubin größer 0 ist; und

l) wenn die Prüfung in Schritt i) ergibt, dass der erste theoretische Extinktionswert $E_{H1}$ für Hämoglobin größer 0 ist, Zuordnen des ersten theoretischen Extinktionswertes $E_{H1}$ für Hämoglobin zu einem von mehreren vorab festgelegten Hämoglobin-Konzentrationsleveln, und

m) wenn die Prüfung in Schritt k) ergibt, dass der erste theoretische Extinktionswert $E_{B1}$ für Bilirubin größer 0 ist, Zuordnen des ersten theoretischen Extinktionswertes $E_{B1}$ für Bilirubin zu einem von mehreren vorab festgelegten Bilirubin-Konzentrationsleveln, und

n) Zuordnen des ersten Messwertes A1 zu einem von mehreren vorab festgelegten Lipid-Konzentrationsleveln.

2. Verfahren nach Anspruch 1, wobei, wenn die Prüfung in Schritt i), ob der erste theoretische Extinktionswert $E_{H1}$ für Hämoglobin größer 0 ist, ergibt, dass er kleiner oder gleich 0 ist, das Fehlen von Hämoglobin festgestellt wird.

3. Verfahren nach Anspruch 1, wobei, wenn die Prüfung in Schritt k), ob der erste theoretische Extinktionswert $E_{B1}$ für Bilirubin größer 0 ist, ergibt, dass er kleiner oder gleich 0 ist, das Fehlen von Bilirubin festgestellt wird.

4. Verfahren nach Anspruch 1, wobei nach der Berechnung der Näherungskurve $L_0$ für die Extinktion E der Lipide, eine korrigierte Näherungskurve $L_k$ für die Extinktion E der Lipide erstellt wird, indem die Näherungskurve $L_0$ mittels eines iterativen Verfahrens korrigiert wird.

5. Verfahren nach Anspruch 4, wobei für die Erstellung der korrigierten Näherungskurve $L_k$

• ein dritter theoretischer Extinktionswert $E_{H3}$ für Hämoglobin bestimmt wird durch Multiplikation des ersten theoretischen Extinktionswertes $E_{H1}$ für Hämoglobin mit einem zweiten vorbestimmten Proportionalitätsfaktor, der das Verhältnis zwischen Extinktionswerten für Hämoglobin bei der dritten Wellenlänge $\lambda3$ und Extinktionswerten für Hämoglobin bei der zweiten Wellenlänge $\lambda2$ widerspiegelt; und

• ein zweiter theoretischer Extinktionswert $E_{B2}$ für Bilirubin bestimmt wird durch Multiplikation des ersten theoretischen Extinktionswertes $E_{B1}$ für Bilirubin mit einem dritten vorbestimmten Proportionalitätsfaktor, der das Verhältnis zwischen Extinktionswerten für Bilirubin bei der zweiten Wellenlänge $\lambda2$ und Extinktionswerten für Bilirubin bei der vierten Wellenlänge $\lambda4$ widerspiegelt; und

• ein erster theoretischer Extinktionswert $E_{L1korr}$ für Lipide bestimmt wird, der der Differenz zwischen dem zweiten Messwert A2 und der Summe des dritten theoretischen Extinktionswertes $E_{H3}$ für Hämoglobin und des zweiten theoretischen Extinktionswertes $E_{B2}$ für Bilirubin entspricht und der erste theoretische Extinktionswert $E_{L1korr}$ zur Berechnung neuer Parameter $q = (\ln A_1 - \ln E_{L1korr})/(\lambda_1 - \lambda_2)$ und $p = E_{L1korr}/e^{\lambda_2 * q}$ und einer korrigierten Näherungskurve $L_k = p * e^{\lambda * q}$ verwendet wird.

6. Verfahren nach Anspruch 4, wobei eine korrigierte Näherungskurve $L_k$ für die Extinktion E der Lipide nur dann erstellt wird, wenn festgestellt wird, dass der Quotient aus der Differenz zwischen dem vierten Messwert A4 und dem Wert der Näherungskurve ($L_0$) bei der vierten Wellenlänge $\lambda4$ und aus dem vierten Messwert A4 größer 0,2 ist.

7. Verfahren nach einem der vorhergehenden Ansprüche, wobei die erste Wellenlänge λ1 im Bereich zwischen 600 nm und 660 nm und die zweite Wellenlänge λ2 im Bereich zwischen 320 nm und 360 nm und die dritte Wellenlänge λ3 im Bereich zwischen 540 nm und 580 nm und die vierte Wellenlänge λ4 im Bereich zwischen 380 nm und 440 nm liegt.

8. Verfahren nach Anspruch 7, wobei die erste Wellenlänge λ1 660 nm und die zweite Wellenlänge λ2 340 nm und die dritte Wellenlänge λ3 575 nm und die vierte Wellenlänge λ4 405 nm beträgt.

9. Verfahren nach einem der vorhergehenden Ansprüche, wobei die Körperflüssigkeitsprobe Blutserum oder Blutplasma ist.

10. Verfahren nach einem der vorhergehenden Ansprüche, wobei das Durchstrahlen der Körperflüssigkeitsprobe mit Licht bei einer Vielzahl von Wellenlängen mit Hilfe von Laser- oder Leuchtdioden oder mit Hilfe einer Lichtquelle mit verschiedenen optischen Filtern erfolgt und wobei das Erfassen der Vielzahl von Messwerten (A1; A2; A3; A4) mit Hilfe eines Photodetektors erfolgt.

11. Automatisches Analysegerät mit einer Messeinrichtung, wobei die Messeinrichtung mindestens eine Lichtquelle und mehrere optische Filter und mindestens einen Photodetektor umfasst, wobei die Messeinrichtung dazu ausgelegt ist, die Verfahrensschritte a) bis e) gemäß Anspruch 1 auszuführen, **dadurch gekennzeichnet, dass** das Analysegerät ferner eine Berechnungseinrichtung umfasst, welche dazu ausgelegt ist, die übrigen Verfahrensschritte zur quantitativen Bestimmung von Lipiden, Hämoglobin und Bilirubin gemäß Anspruch 1 auszuführen.

12. Automatisches Analysegerät mit einer Messeinrichtung, wobei die Messeinrichtung mehrere Lichtquellen, vorzugsweise mehrere Leucht- oder Laserdioden, und mindestens einen Photodetektor umfasst, wobei die Messeinrichtung dazu ausgelegt ist, die Verfahrensschritte a) bis e) gemäß Anspruch 1 auszuführen, **dadurch gekennzeichnet, dass** das Analysegerät ferner eine Berechnungseinrichtung umfasst, welche dazu ausgelegt ist, die übrigen Verfahrensschritte zur quantitativen Bestimmung von Lipiden, Hämoglobin und Bilirubin gemäß Anspruch 1 auszuführen.

13. Automatisches Analysegerät gemäß Anspruch 11 oder Anspruch 12, wobei die Messeinrichtung mindestens vier Lichtquellen umfasst, wobei die erste Lichtquelle Licht einer Wellenlänge im Bereich zwischen 600 nm und 660 nm emittiert, und die zweite Lichtquelle Licht einer Wellenlänge im Bereich zwischen 320 nm und 360 nm emittiert, und die dritte Lichtquelle Licht einer Wellenlänge im Bereich zwischen 540 nm und 580 nm emittiert, und die vierte Lichtquelle Licht einer Wellenlänge im Bereich zwischen 380 nm und 440 nm emittiert.

14. Automatisches Analysegerät gemäß Anspruch 13, wobei die erste Lichtquelle Licht einer Wellenlänge von 660 nm emittiert, und die zweite Lichtquelle Licht einer Wellenlänge von 340 nm emittiert, und die dritte Lichtquelle Licht einer Wellenlänge von 575 nm emittiert, und die vierte Lichtquelle Licht einer Wellenlänge von 405 nm emittiert.

**Claims**

1. Method for quantitatively determining lipids, haemoglobin and bilirubin in a body fluid sample, comprising the steps of:

   a) transirradiating the body fluid sample with light at a multiplicity of wavelengths λ;
   b) capturing a first measurement value A1 at a first wavelength λ1 at which the absorbance not caused by lipids is negligible;
   c) capturing a second measurement value A2 at a second wavelength λ2 at which the absorbance not caused by bilirubin and haemoglobin and lipids is negligible;
   d) capturing a third measurement value A3 at a third wavelength λ3 at which haemoglobin has an absorbance maximum;
   e) capturing a fourth measurement value A4 at a fourth wavelength λ4 at which the absorbance not caused by bilirubin and haemoglobin and lipids is negligible;
   f) determining a parameter q using the formula:

$$q = \frac{\ln A1 - \ln A2}{\lambda 1 - \lambda 2}$$

and a parameter p using the formula:

$$p = \frac{A2}{e^{\lambda 2 \cdot q}}$$

g) calculating an exponential approximation curve ($L_0$) of the form:

$$E = f(\lambda) = p \cdot e^{\lambda \cdot q}$$

for the absorbance E of the lipids;

h) determining a first theoretical absorbance value $E_{H1}$ for haemoglobin, corresponding to the difference between the third measurement value A3 and the value of the approximation curve ($L_0$) at the third wavelength $\lambda3$;

i) checking whether the first theoretical absorbance value $E_{H1}$ for haemoglobin is greater than 0 and, if it is greater than 0, determining a second theoretical absorbance value $E_{H2}$ for haemoglobin by multiplying the first theoretical absorbance value $E_{H1}$ for haemoglobin by a first predetermined proportionality factor which reflects the ratio between absorbance values for haemoglobin at the third wavelength $\lambda3$ and absorbance values for haemoglobin at the fourth wavelength $\lambda4$;

j) determining a first theoretical absorbance value $E_{B1}$ for bilirubin, corresponding to the difference between the fourth measurement value A4 and the sum of the value of the approximation curve ($L_0$) at the fourth wavelength $\lambda4$ and of the second theoretical absorbance value $E_{H2}$ for haemoglobin;

k) checking whether the first theoretical absorbance value $E_{B1}$ for bilirubin is greater than 0; and

l) if the check in step i) reveals that the first theoretical absorbance value $E_{H1}$ for haemoglobin is greater than 0, assigning the first theoretical absorbance value $E_{H1}$ for haemoglobin to one of multiple predefined haemoglobin concentration levels, and

m) if the check in step k) reveals that the first theoretical absorbance value $E_{B1}$ for bilirubin is greater than 0, assigning the first theoretical absorbance value $E_{B1}$ for bilirubin to one of multiple predefined bilirubin concentration levels, and

n) assigning the first measurement value A1 to one of multiple predefined lipid concentration levels.

2. Method according to Claim 1, wherein, if the check in step i) to determine whether the first theoretical absorbance value $E_{H1}$ for haemoglobin is greater than 0 reveals that said value is less than or equal to 0, the absence of haemoglobin is established.

3. Method according to Claim 1, wherein, if the check in step k) to determine whether the first theoretical absorbance value $E_{B1}$ for bilirubin is greater than 0 reveals that said value is less than or equal to 0, the absence of bilirubin is established.

4. Method according to Claim 1, wherein the calculation of the approximation curve $L_0$ for the absorbance E of the lipids is followed by the creation of a corrected approximation curve $L_c$ for the absorbance E of the lipids, by correcting the approximation curve $L_0$ by means of an iterative method.

5. Method according to Claim 4, wherein, for the creation of the corrected approximation curve $L_c$,

• a third theoretical absorbance value $E_{H3}$ for haemoglobin is determined by multiplying the first theoretical absorbance value $E_{H1}$ for haemoglobin by a second predetermined proportionality factor which reflects the ratio between absorbance values for haemoglobin at the third wavelength $\lambda3$ and absorbance values for haemoglobin at the second wavelength $\lambda2$; and

• a second theoretical absorbance value $E_{B2}$ for bilirubin is determined by multiplying the first theoretical absorbance value $E_{B1}$ for bilirubin by a third predetermined proportionality factor which reflects the ratio between absorbance values for bilirubin at the second wavelength $\lambda2$ and absorbance values for bilirubin at the fourth wavelength $\lambda4$; and

• a first theoretical absorbance value $E_{L1corr}$ for lipids is determined, corresponding to the difference between the second measurement value A2 and the sum of the third theoretical absorbance value $E_{H3}$ for haemoglobin and of the second theoretical absorbance value $E_{B2}$ for bilirubin, and the first theoretical absorbance value $E_{L1corr}$ is used to calculate new parameters q = (ln $A_1$ - ln $E_{L1corr}$) / ($\lambda_1$ - $\lambda_2$) and p = $E_{L1corr}$ / $e^{\lambda_2 * q}$ and a corrected approximation curve $L_c$ = p*$e^{\lambda * q}$.

**6.** Method according to Claim 4, wherein a corrected approximation curve $L_c$ for the absorbance E of the lipids is only created when it is established that the quotient formed from the difference between the fourth measurement value A4 and the value of the approximation curve ($L_0$) at the fourth wavelength $\lambda4$ and from the fourth measurement value A4 is greater than 0.2.

**7.** Method according to any of the preceding claims, wherein the first wavelength $\lambda1$ is within the range between 600 nm and 660 nm and the second wavelength $\lambda2$ is within the range between 320 nm and 360 nm and the third wavelength $\lambda3$ is within the range between 540 nm and 580 nm and the fourth wavelength $\lambda4$ is within the range between 380 nm and 440 nm.

**8.** Method according to Claim 7, wherein the first wavelength $\lambda1$ is 660 nm and the second wavelength $\lambda2$ is 340 nm and the third wavelength $\lambda3$ is 575 nm and the fourth wavelength $\lambda4$ is 405 nm.

**9.** Method according to any of the preceding claims, wherein the body fluid sample is blood serum or blood plasma.

**10.** Method according to any of the preceding claims, wherein the transirradiation of the body fluid sample with light at a multiplicity of wavelengths is achieved using laser or light-emitting diodes or using a light source having various optical filters and wherein the capture of the multiplicity of measurement values (A1; A2; A3; A4) is achieved using a photodetector.

**11.** Automatic analyser comprising a measuring device, said measuring device comprising at least one light source and multiple optical filters and at least one photodetector, said measuring device being designed to carry out method steps a) to e) according to Claim 1, **characterized in that** the analyser further comprises a calculation device designed to carry out the remaining method steps according to Claim 1 for quantitatively determining lipids, haemoglobin and bilirubin.

**12.** Automatic analyser comprising a measuring device, said measuring device comprising multiple light sources, preferably multiple light-emitting or laser diodes, and at least one photodetector, said measuring device being designed to carry out method steps a) to e) according to Claim 1, **characterized in that** the analyser further comprises a calculation device designed to carry out the remaining method steps according to Claim 1 for quantitatively determining lipids, haemoglobin and bilirubin.

**13.** Automatic analyser according to Claim 11 or Claim 12, wherein the measuring device comprises at least four light sources, the first light source emitting light of a wavelength within the range between 600 nm and 660 nm, and the second light source emitting light of a wavelength within the range between 320 nm and 360 nm, and the third light source emitting light of a wavelength within the range between 540 nm and 580 nm, and the fourth light source emitting light of a wavelength within the range between 380 nm and 440 nm.

**14.** Automatic analyser according to Claim 13, wherein the first light source emits light of a wavelength of 660 nm, and the second light source emits light of a wavelength of 340 nm, and the third light source emits light of a wavelength of 575 nm, and the fourth light source emits light of a wavelength of 405 nm.

**Revendications**

**1.** Procédé de détermination quantitative de lipides, de l'hémoglobine et de la bilirubine dans un échantillon de liquide corporel, comprenant les stades :

a) on expose l'échantillon de liquide corporel à de la lumière ayant une pluralité de longueurs d'onde $\lambda$ ;
b) on relève une première valeur A1 de mesure à une première longueur d'onde $\lambda1$, à laquelle l'extinction, qui n'est pas provoquée par des lipides, est négligeable ;
c) on relève une deuxième valeur A2 de mesure à une deuxième longueur d'onde $\lambda2$, à laquelle l'extinction, qui n'est pas provoquée par la bilirubine et l'hémoglobine et des lipides, est négligeable ;
d) on relève une troisième valeur A3 de mesure à une troisième longueur d'onde A3, à laquelle l'hémoglobine présente un maximum d'extinction ;
e) on relève une quatrième valeur A4 de mesure à une quatrième longueur d'onde A4, à laquelle l'extinction, qui n'est pas provoquée par la bilirubine et l'hémoglobine et des lipides, est négligeable ;
f) on détermine un paramètre q par la formule :

$$q = \frac{InA1 - InA2}{\lambda 1 - \lambda 2}$$

et un paramètre p par la formule :

$$p = \frac{A2}{e^{\lambda 2.q}}$$

g) on calcule une courbe ($L_0$) approchée exponentielle de la forme :

$$E = f(\lambda) = p.e^{\lambda.q}$$

pour l'extinction des lipides ;

h) on détermine une première valeur $E_{H1}$ théorique d'extinction pour l'hémoglobine, qui correspond à la différence entre la troisième valeur A3 de mesure et la valeur de la courbe ($L_0$) approchée à la troisième longueur d'onde A3 ;

i) on contrôle si la première valeur $E_{H1}$ théorique d'extinction pour l'hémoglobine est plus grande que 0 et si elle est plus grande que 0, on détermine une deuxième valeur $E_{H2}$ théorique d'extinction pour l'hémoglobine en multipliant la première valeur $E_{H1}$ théorique d'extinction pour l'hémoglobine par un premier facteur de proportionnalité déterminé à l'avance, qui reflète le rapport entre des valeurs d'extinction pour l'hémoglobine à la troisième longueur d'onde A3 et des valeurs d'extinction pour l'hémoglobine à la quatrième longueur d'onde A4 ;

j) on détermine une première valeur $E_{B1}$ théorique d'extinction pour la bilirubine, qui correspond à la différence entre la quatrième valeur A4 de mesure et la somme de la valeur de la courbe ($L_0$) approchée à la quatrième longueur d'onde $\lambda 4$ et de la deuxième valeur $E_{H2}$ théorique d'extinction pour l'hémoglobine ;

k) on contrôle si la première valeur $E_{B1}$ théorique d'extinction pour la bilirubine est plus grande que 0 ; et

l) si le contrôle au stade i) donne que la première valeur $E_{H1}$ théorique d'extinction pour l'hémoglobine est plus grande que 0, on affecte la première valeur $E_{H1}$ théorique d'extinction pour l'hémoglobine à l'un de plusieurs niveaux de concentration d'hémoglobine fixés à l'avance, et

m) si le contrôle au stade k) donne que la première valeur $E_{B1}$ théorique d'extinction pour la bilirubine est plus grande que 0, on affecte la première valeur $E_{B1}$ théorique d'extinction pour la bilirubine à l'un de plusieurs niveaux de concentration de bilirubine fixés à l'avance, et

n) on affecte la première valeur A1 de mesure à l'un de plusieurs niveaux de concentration de lipides fixés à l'avance.

2. Procédé suivant la revendication 1, dans lequel, si le contrôle au stade i) du point de savoir si la première valeur $E_{H1}$ théorique d'extinction pour l'hémoglobine est plus grande que 0 donne qu'elle est inférieure ou égale à 0, on constate l'absence d'hémoglobine.

3. Procédé suivant la revendication 1, dans lequel, si le contrôle au stade k) sur le point de savoir si la première valeur $E_{B1}$ théorique d'extinction pour la bilirubine est plus grande que 0 donne qu'elle est inférieure ou égale à 0, on constate l'absence de bilirubine.

4. Procédé suivant la revendication 1, dans lequel, après le calcul de la courbe $L_0$ approchée de l'extinction E des lipides, on établit une courbe $L_k$ approchée corrigée de l'extinction E des lipides en corrigeant la courbe $L_0$ approchée au moyen d'un procédé par itération.

5. Procédé suivant la revendication 4, dans lequel, pour l'établissement de la courbe $L_k$ approchée corrigée

• on détermine une troisième valeur $E_{H3}$ théorique d'extinction pour l'hémoglobine en multipliant la première valeur $E_{H1}$ théorique d'extinction pour l'hémoglobine par un deuxième facteur de proportionnalité déterminé à l'avance, qui reflète le rapport entre des valeurs d'extinction pour l'hémoglobine à la troisième longueur d'onde $\lambda 3$ et des valeurs d'extinction pour l'hémoglobine à la deuxième longueur d'onde $\lambda 2$ ; et

• on détermine une deuxième valeur EB2 théorique d'extinction pour la bilirubine en multipliant la première valeur $E_{B1}$ théorique d'extinction pour la bilirubine par un troisième facteur de proportionnalité déterminé à l'avance, qui reflète le rapport entre des valeurs d'extinction pour la bilirubine à la deuxième longueur d'onde $\lambda 2$ et des valeurs d'extinction pour la bilirubine à la quatrième longueur d'onde $\lambda 4$ ; et

- on détermine une première valeur $E_{l1korr}$ théorique d'extinction pour des lipides, qui correspond à la différence entre la deuxième valeur A2 de mesure et la somme de la troisième valeur $E_{H3}$ théorique d'extinction pour l'hémoglobine et la deuxième valeur EB2 théorique d'extinction pour la bilirubine et on utilise la première valeur $E_{l1korr}$ théorique d'extinction pour le calcul de nouveaux paramètres q= (ln A$_1$-ln $E_{l1korr}$)/($\lambda_1$-$\lambda_2$) et p= $E_{l1korr}$/e$^{\lambda2*q}$ et d'une courbe Lk=p*e$^{\lambda*q}$ approchée corrigée.

6. Procédé suivant la revendication 4, dans lequel on n'établit une courbe $L_k$ approchée corrigée de l'extinction E des lipides que si l'on constate que le quotient de la différence entre la quatrième valeur A4 de mesure et la valeur de la courbe ($L_0$) approchée à la quatrième longueur d'onde $\lambda4$ par la quatrième valeur A4 de mesure est plus grand que 0,2.

7. Procédé suivant l'une des revendications précédentes, dans lequel la première longueur d'onde $\lambda1$ est dans la plage comprise entre 600 nm et 660 nm et la deuxième longueur d'onde $\lambda2$ dans la plage comprise entre 320 nm et 360 nm et la troisième longueur d'onde $\lambda3$ dans la plage comprise entre 540 nm et 580 nm et la quatrième longueur d'onde $\lambda4$ dans la plage comprise entre 380 nm et 440 nm.

8. Procédé suivant la revendication 7, dans lequel la première longueur d'onde $\lambda1$ est de 660 nm et la deuxième longueur d'onde $\lambda2$ de 340 nm et la troisième longueur d'onde $\lambda3$ de 575 nm et la quatrième longueur d'onde $\lambda4$ de 405 nm

9. Procédé suivant l'une des revendications précédentes, dans lequel l'échantillon de liquide corporel est du sérum sanguin ou du plasma sanguin.

10. Procédé suivant l'une des revendications précédentes, dans lequel l'exposition de l'échantillon de liquide corporel à de la lumière ayant une pluralité de longueurs d'onde s'effectue à l'aide de diodes laser ou électroluminescentes ou à l'aide d'une source de lumière ayant des filtres optiques différents et le relevé de la pluralité de valeurs (A1 ; A2 ; A3 ; A4) de mesure s'effectue à l'aide d'un photodétecteur.

11. Appareil d'analyse automatique comprenant un dispositif de mesure, le dispositif de mesure comprenant au moins une source de lumière et plusieurs filtres optiques et au moins un photodétecteur, le dispositif de mesure étant constitué pour effectuer les stades a) à e) de procédé suivant la revendication 1, **caractérisé en ce que** l'appareil d'analyse comprend, en outre, un dispositif de calcul conçu pour effectuer les autres stades de procédé de détermination quantitative de lipides, de l'hémoglobine et de la bilirubine suivant la revendication 1.

12. Appareil d'analyse automatique comprenant un dispositif de mesure, le dispositif de mesure comprenant plusieurs sources de lumière, de préférence plusieurs diodes électroluminescentes ou laser, et au moins un photodétecteur, la dispositif de mesure étant conçu pour effectuer les stades a) à e) de procédé suivant la revendication 1, **caractérisé en ce que** l'appareil d'analyse comprend, en outre, un dispositif de calcul conçu pour effectuer les autres stades de procédé de détermination quantitative de lipides, de l'hémoglobine et de la bilirubine suivant la revendication 1.

13. Appareil d'analyse automatique suivant la revendication 11 ou la revendication 12, le dispositif de mesure comprenant au moins quatre sources de lumière, la première source de lumière émettant de la lumière d'une longueur d'onde dans la plage comprise entre 600 nm et 660 nm, et la deuxième source de lumière émettant de la lumière d'une longueur d'onde dans la plage comprise entre 320 nm et 360 nm, et la troisième source de lumière émettant de la lumière d'une longueur d'onde dans la plage comprise entre 540 nm et 580 nm, et la quatrième source de lumière émettant de la lumière d'une longueur d'onde dans la plage comprise entre 380 nm et 440 nm.

14. Appareil d'analyse automatique suivant la revendication 13, dans lequel la première source de lumière émet de la lumière d'une longueur d'onde de 660 nm, et la deuxième source de lumière émet de la lumière d'une longueur d'onde de 340 nm, et la troisième source de lumière émet de la lumière d'une longueur d'onde de 575 nm, et la quatrième source de lumière émet de la lumière d'une longueur d'onde de 45 nm.

**IN DER BESCHREIBUNG AUFGEFÜHRTE DOKUMENTE**

**In der Beschreibung aufgeführte Patentdokumente**

- EP 1059522 A1
- US 4263512 A
- US 20090009750 A1
- US 20100174491 A1
- WO 2013010970 A1
- EP 3051272 A2